# EUROPEAN PATENT SPECIFICATION

(11) **EP 2 666 461 B1**
(45) Date of publication and mention of the grant of the patent: **01.07.2015**
(21) Application number: 12382191.0
(22) Date of filing: 23.05.2012
(51) Int. Cl.: A61K 31/573, A61K 31/575, A61K 31/704, A61K 36/42, A61Q 5/02, A61K 8/63, A61Q 7/00

(54) **Terpene extract for the treatment of hair loss**
Terpenextrakt zur Behandlung von Haarausfall
Extrait de terpène pour le traitement de la perte des cheveux

(43) Date of publication of application: 27.11.2013
(73) Proprietor: Tergum S.L., 41702 Dos Hermanas, Sevilla (ES)
(72) Inventor: Rodríguez Marín, Carlos Luis, 41702 Sevilla (ES)
(74) Representative: Hoffmann Eitle

(56) References cited:
- WO-A1-2010/035299
- US-A- 5 925 356
- GREIGE-GERGES ET AL: "Cucurbitacins from Ecballium elaterium juice increase the binding of bilirubin and ibuprofen to albumin in human plasma", CHEMICO-BIOLOGICAL INTERACTIONS, ELSEVIER SCIENCE IRLAND, IR, vol. 169, no. 1, 13 July 2007 (2007-07-13) , pages 53-62, XP022152868, ISSN: 0009-2797, DOI: 10.1016/J.CBI.2007.05.003
- DATABASE WPI Week 200669 Thomson Scientific, London, GB; AN 2006-662383 XP002688880, & JP 2006 206537 A (KOSE KK) 10 August 2006 (2006-08-10)

## Description

### Field of the Invention

The present invention relates to compositions comprising cucurbitacins for use in the treatment and prevention of hair loss conditions and disorders.

### Background of the invention

The following discussion on the background of the invention is merely provided to aid the reader in understanding the invention and is not admitted to describe or constitute prior art to the present invention.

Hair loss is an extremely prevalent condition and it is estimated that approximately 20 to 25% of the population suffer from some type of hair loss disorder. Whilst hair loss is more common in adult males, hair loss disorders also affect women and children. Although most hair loss disorders are not in themselves damaging to the health of an individual, many sufferers report associated psychological problems including anxiety and depression.

Despite the prevalence of hair loss disorders, few efficacious treatments are available. Surgical treatments are available for the restoration of hair, particularly to the scalp. However, these techniques are painful, expensive, and suffer from the inherent dangers associated with any invasive surgery. Furthermore, hair restoration surgeries typically require at least some areas of dense hair growth to be available on the patient for transplant or relocation to other areas of the body. Yet further, surgical treatments are rarely applicable to sufferers of alopecia areata in which spontaneous regrowth of hair which subsequently falls out again often occurs.

Hair loss compositions are thus an attractive proposition for those suffering from hair loss conditions. A range of therapies are available for which partial success in the treatment of alopecia has been claimed. The various therapies available can be divided up into several groups comprising non-specific immunosuppressants such as corticosteroids or UVA treatment, contact dermatitis inducers, specific immunosuppressants such as cyclosporins, non-specific irritants and other treatments such as alternative and experimental therapies.

Non-specific immunosuppressants such as corticosteroids function by mimicking the steroidal hormones produced by the adrenal glands to suppress inflammation. Corticosteroids can be topically administered to the affected area, through intralesional injections, or systemically via injection or oral medication, depending on the severity of the condition. While topical corticosteroids are commonly used to treat alopecia areata, there is little evidence that they promote hair regrowth. Intralesional administration of corticosteroids has demonstrated more efficacy in the treatment of alopecia areata, with a study reported by Porter and Burton (Br. J. Dermatol. 1971:85, 272-273) demonstrating that hair regrowth was achieved in 33 out of 34 sites injected with triamcinolone hexacetonide in 11 patients and in 16 out of 25 sites injected with triamcinolone acetonide in 17 patients, with the affects lasting about nine months. However, intralesional corticosteroid administration is most suitable for treating patchy hair loss of limited extent and for cosmetically sensitive sites such as the eyebrows, with skin atrophy at the site of injection a consistent side-effect. Systemic corticosteroid administration has achieved some hair regrowth in alopecia suffers. However, studies have demonstrated only 30-47% of patients showed significant hair regrowth and thus in most patients the response achieved is insufficient to justify the risks associated with prolonged treatment.

Contact dermatitis inducers work by sensitising the immune system. A low level of the drug is initially applied and adjusted until a reaction is established. Contact dermatitis inducers utilised for the treatment of alopecia areata include dinitrochlorobenzene (DNCB), diphenylcyclopropenone (DPCP) and squaric-acid-dibutylester (SADBE). Reviews of these treatments have concluded that the range of response rates is wide, at reportedly 9 to 87% (Rokhsar et al. J Am Acad Dermatol 1998:39:751-761). Side effects including severe dermatitis and occipital and/or cervical lymphadenopathy are often common.

Specific immunosuppressants for the treatment of alopecia include cyclosporins and tacrolimus (FK506). These drugs act by inhibiting T-cell activation. While results have demonstrated some efficacy for cyclosporins, side-effects are a major consideration and results may often not justify the associated risks.

Alternative treatments such as essential oils and acupuncture and experimental and theoretical treatments such as cytokines, biologicals, desensitisation, oral tolerance and gene therapy have been variously employed in the treatment of alopecia with varying degrees of success.

Therefore, despite active research in this area and a large number of proposed active ingredients there remains the need for a hair loss composition which demonstrates efficacy in a wide populace with minimal side effects.

### Brief description of the invention

A first aspect of the present invention refers to a composition (from hereinafter composition of the invention) comprising cucurbitacins and their glucoside derivatives, wherein cucurbitacins D, I, L and A and their glucoside derivatives are present, as a whole, in terms of percentage by weight, in an amount greater than 25% of the total weight of the cucurbitacins and their glucoside derivatives, and wherein cucurbitacins B, E and the derivative of cucurbitacin E, ecballic acid, are each present at a concentration below 2.5% of the total weight of the cucurbitacins and their glucoside derivatives.

In a preferred embodiment of the present invention, cucurbitacins D, I, L and A and their glucoside derivatives are present, as a whole, in terms of percentage by weight, in an amount greater than 30% of the total weight of the cucurbitacins and their glucoside derivatives, more preferably in an amount greater than 35%, 40% or 45%.

In a more preferred embodiment of the present invention, cucurbitacins D, I, L and A and their glucoside derivatives are present, as a whole, in terms of percentage by weight in an amount between 25 to 50% of the total weight of the cucurbitacins and their glucoside derivatives.

In a still more preferred embodiment of the invention, the composition of the invention further comprises:
a. Cucurbitacin O;
b. Cucurbitacin P;
c. Cucurbitacin C;
d. Cucurbitacin H;
e. Cucurbitacin J;
f. Cucurbitacin F; or
g. Cucurbitacin Q;
wherein each of these compounds is present in an amount greater than 2.5% of the total weight of the cucurbitacins and their glucoside derivatives.

In a still more preferred embodiment of the invention, the composition of the invention further comprises olive oil.

A second aspect of the invention refers to a vegetable extract obtained from Ecbalium Elaterium wherein said vegetable extract comprises cucurbitacins and/or their glucoside derivatives, wherein cucurbitacins D, I, L and A and/or their glucoside derivatives are present, as a whole, in terms of percentage by weight, in an amount greater than 25% of the total weight of the cucurbitacins and their glucoside derivatives, and wherein cucurbitacins B, E and the derivative of cucurbitacin E, ecballic acid, are each present at a concentration below 2.5% of the total weight of the cucurbitacins and their glucoside derivatives.

A third aspect of the invention refers to a composition (from hereinafter composition of the third aspect of the invention) comprising cucurbitacins and/or their glucoside derivatives, wherein cucurbitacins D, I, L and A and their glucoside derivatives are present, as a whole, in terms of percentage by weight, in an amount greater than 25% of the total weight of the cucurbitacins and their glucoside derivatives, and wherein cucurbitacins B, E and the derivative of cucurbitacin E, ecballic acid, are each present at a concentration below 2.5% of the total weight of the cucurbitacins and their glucoside derivatives, wherein this composition is obtainable by a leaching process comprising the steps of:
a. Obtaining a portion of a plant pertaining to the *cucurbitaceae* family and
b. extracting the vegetable cucurbitacins from said portion by a heating process and by using a liquid extractant capable of solubilising said compounds.

In a preferred embodiment of this aspect of the invention, the plant portion pertaining to the *cucurbitaceae* family is Ecballium elaterium.

In another embodiment of this aspect of the invention, the heating process is conducted at a temperature from 120 C° to 180 C°, preferably at a temperature of approximately 150 C°.

In another embodiment of this aspect of the invention, the plant portion is selected from the group consisting of: roots, stems, leaves or fruits or any combination thereof. Preferably the plant portion is a root.

In a still further embodiment of this aspect of the invention, the extractant is olive oil, wherein preferably the extractant is enriched with natural or added content of antioxidants.

A fourth aspect of the invention refers to the composition of any of the previous aspects for its use in therapy.

A fifth aspect of the invention refers to the composition of any of the previous aspects for its use in the treatment or prevention of hair loss. In a preferred embodiment of the invention, hair loss is caused by hormonal deregulations, androgenic alopecia or from alopecia of seborrheic origin.

In a preferred embodiment, the composition of any of the previous aspects is use as a cosmetic composition.

A further aspect of the invention refers to a process for the preparation of a composition comprising cucurbitacins and/or their glucoside derivatives, wherein cucurbitacins D, I, L and A and their glucoside derivatives are present, as a whole, in terms of percentage by weight, in an amount greater than 25% of the total weight of the cucurbitacins and their glucoside derivatives, and wherein cucurbitacins B, E and the derivative of cucurbitacin E, ecballic acid, are each present at a concentration below 2.5% of the total weight of the cucurbitacins and their glucoside derivatives, which comprises the following steps:
a. Obtaining a portion of a plant pertaining to the *cucurbitaceae* family and
b. extracting the vegetable cucurbitacins from said portion by a heating process and by using a liquid extractant capable of solubilising said compounds.

In a preferred embodiment of this aspect of the invention, the plant portion pertaining to the *cucurbitaceae* family is Ecballium elaterium.

In another embodiment of this aspect of the invention, the heating process is conducted at a temperature from 120 C° to 180 C°, preferably at a temperature of approximately 150 C°.

In another embodiment of this aspect of the invention, the plant portion is selected from the group consisting of: roots, stems, leaves or fruits or any combination thereof. Preferably the plant portion is a root.

In a still further embodiment of this aspect of the invention, the extractant is olive oil, wherein preferably the extractant is enriched with natural or added content of antioxidants.

A sixth aspect of the present invention refers to a shampoo composition (from hereinafter shampoo composition of the invention) comprising:
a. A cleansing base; and
b. Active ingredients,
wherein at least one of the active ingredients comprises the composition as defined in any of the previous aspects.

In a preferred embodiment of this aspect of the invention, the cleansing base comprises surfactants of the types: alkyl sulphates and/or an alkyl ether sulphates.

In another embodiment of this aspect of the invention, the shampoo composition of the invention further comprises one or more of the following components or any combination thereof:
a. Thickeners for surfactant preparation;
b. Pearlescents and opacifiers;
c. Perfumes;
d. Colorants; or
e. Stabilizing agents.

In a further embodiment of this aspect of the invention, the shampoo composition of the invention is an anti-dandruff composition comprising active ingredients capable of re-establishing the natural microbial flora on the skin surface.

In a further embodiment of this aspect of the invention, the shampoo composition of the invention is a shampoo for greasy hair comprising a cleansing base capable of eliminating excess sebum.

In a further embodiment of this aspect of the invention, the shampoo composition of the invention is for sensitive scalp and comprises mild anionic surphactants and amphoterics and/or non-ionic surfactants.

In a still further embodiment of this aspect of the invention, the shampoo composition of the invention is for dry hair and comprises:
a. A fat absorber; and/or
b. An abrasive to dislodge soil; and/or
c. An alkalizer

A seventh aspect of the present invention refers to a hair lotion (from hereinafter lotion of the invention) comprising the composition as defined in any of the first, second or third aspects of the present invention.

In a preferred embodiment of this aspect of the invention, the lotion is in the form of liquid, gel, cream, an emulsion, oil or serum.

An eighth aspect of the present invention refers to the shampoo of the invention or to the lotion of the invention, for its use in the treatment or prevention of hair loss.

A ninth aspect of the present invention refers to a pharmaceutical composition (from hereinafter the pharmaceutical composition of the invention) comprising the composition as defined in any of the first, second or third aspects of the present invention for its use in therapy. Preferably, the pharmaceutical composition of the invention is use in the treatment or prevention of hair loss. More preferably, the pharmaceutical composition of the invention is use in the treatment or prevention of hair loss caused by hormonal deregulations, androgenic alopecia or from alopecia of seborrheic origin.

A preferred embodiment of this aspect of the invention refers to the pharmaceutical composition of the invention further comprising a pharmaceutically acceptable excipient.

In a preferred embodiment of this aspect of the invention the pharmaceutical composition of the invention comprises a vehicle suitable for topical administration.

In a still further embodiment of this aspect of the invention the pharmaceutical composition of the invention comprises a vehicle suitable for intradermal administration.

### Brief description of the figures

Fig. 1: Figure 1 illustrates a leaching process for the production of the composition of the invention.
Fig. 2: Figure 2 illustrates the i) initial state, ii) evolution after treatment for 6 six months and iii) final results obtained after one year of treatment, for the treatment of Example 4.
Fig. 3: Figure 3 illustrates the results before treatment and after treatment for a period of 6 months, for the treatment of Example 5.
Fig. 4: Figure 4 illustrates the results before and after treatment for the treatment of Example 6.

### Description of the invention

### Definitions

For the following defined terms, these definitions shall be applied, unless a different definition is given in the claims or elsewhere in this specification.

The terms "cucurbitacin", "cucurbitacin compound", and "cucurbitacin derivative" are used interchangeably in the specification to mean a group of triterpenoid substances which are structurally characterized by the tetracyclic cucurbitane nucleus skeleton, namely, 19-(10->9beta)-abeo-10alpha-lanost-5-ene (also known as 9beta-methyl-19-norlanosta-5-ene), with a variety of substitutions at different positions of the structure such as H, O, hydroxyl, alkyl, alkenyl, alkynyl, halogen, alkoxy, aryl and heteroaryl.

The term "cucurbitacin D" is used in the specification to mean compounds having the following chemical formula:

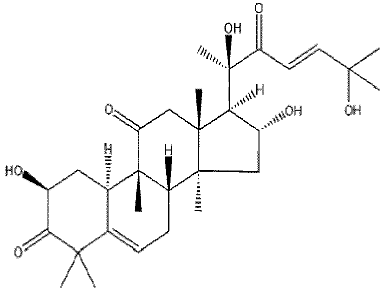

The term "cucurbitacin I" is used in the specification to mean compounds having the following chemical formula:

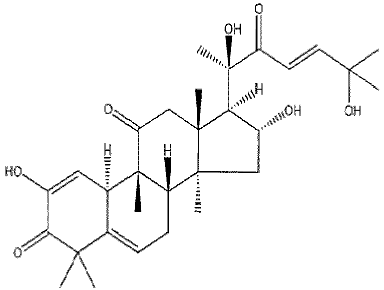

The term "cucurbitacin L" is used in the specification to mean compounds having the following chemical formula:

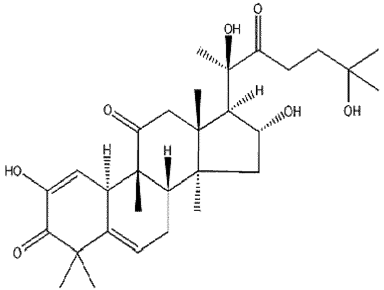

The term "cucurbitacin D" is used in the specification to mean compounds having the following chemical formula:

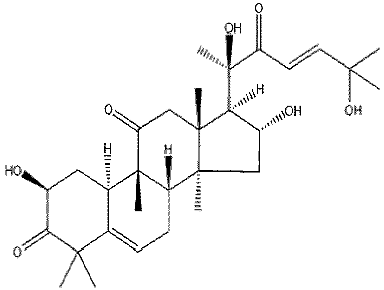

The term "cucurbitacin O" is used in the specification to mean compounds having the following chemical formula:

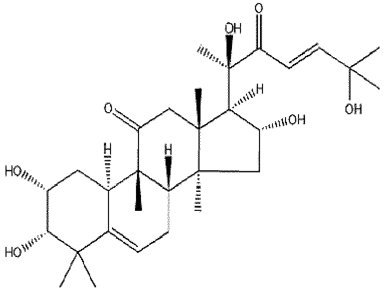

The term "cucurbitacin P" is used in the specification to mean compounds having the following chemical formula:

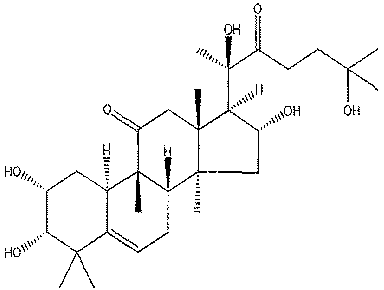

The term "cucurbitacin C" is used in the specification to mean compounds having the following chemical formula:

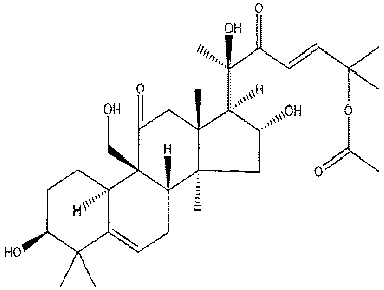

The term "cucurbitacin H" is used in the specification to mean compounds having the following chemical formula:

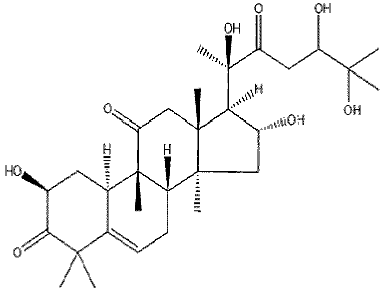

The term "cucurbitacin J" is used in the specification to mean compounds having the following chemical formula:

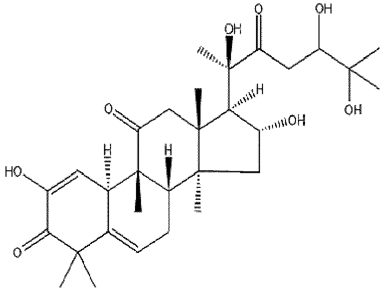

The term "cucurbitacin F" is used in the specification to mean compounds having the following chemical formula:

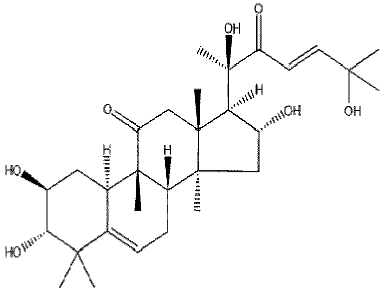

The term "cucurbitacin Q" is used in the specification to mean compounds having the following chemical formula:

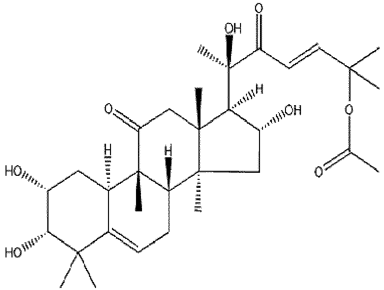

The term "ecballic acid" is used in the specification to mean compounds having the following chemical formula:

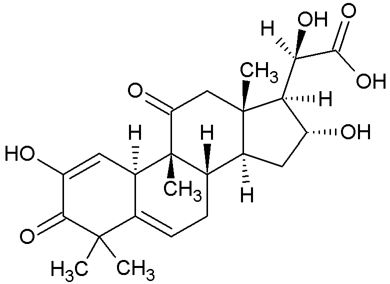

The term "cucurbitacin glucoside" is used in the specification to mean cucurbitacin derivatives formed by condensation of cucurbitacins with glucose units at the hydroxyl positions. The formation of glucoside derivatives is common in plant metabolisms as a pathway destined to store secondary metabolites involved in defense mechanisms in tissues or as a strategy to increase solubility of non-polar metabolites.

The term "total weight of the cucurbitacins and their glucoside derivatives" is used in the specification to mean the total concentration of cucurbitacins and glucoside derivatives measured by a chromatographic method such as that reported by Greige-Gerges et al. (Chemico-Biological Interactions 169 (2007) 53-62). This method is based on the analysis of the extract by liquid chromatography with mass spectrometry detection (LC-MS) providing a chromatographic peak signal for each cucurbitacin and glucoside derivative (limit of detection 0.5-1.0 mg/L). The peak area of the signal associated to each cucurbitacin or derivative is a function of its concentration in the extract. Quantitation of each cucurbitacin or derivative is carried out by preparation of a calibration model using the same analytical protocol with standard solutions of cucurbitacin E at different concentrations. This model enables to obtain a calibration curve by plotting peak area of cucurbitacin E chromatographic signals versus cucurbitacin E concentration in the solutions used to prepare the calibration model. The interpolation in the calibration curve of the peak area for each cucurbitacin or derivative provided by analysis of the extract enables to estimate the concentration of each cucurbitacin or derivative expressed relatively as cucurbitacin E. The total concentration of cucurbitacins and derivatives is calculated by summing up the concentrations of individual cucurbitacins and derivatives. The total concentration of cucurbitacins and derivatives is expressed as percentage concentration (total weight of the cucurbitacins and their glucoside derivatives) referred to the total weight of plant material used for extraction. As exposed above, cucurbitacins D, I, L and A and their glucoside derivatives should be present in the extract as percentage by weight, in an amount greater than 25% of the total weight of the cucurbitacins and their glucoside derivatives measured by the test described in this paragraph. On the other hand, cucurbitacins B, E and the derivative of cucurbitacin E, ecballic acid, are each present at a concentration below 2.5% of the total weight of the cucurbitacins and their glucoside derivatives.

As used herein, "pharmaceutically acceptable carrier" means any material which, when combined with the composition of the invention, allows the compound to retain biological activity.

The term "alopecia" as used herein is taken to mean a disease in which the hair falls out and includes, but is not limited to alopecia areata, male and female pattern baldness, alopecia androgenetica, alopecia totalis, alopecia universalis, alopecia partialis, alopecia barbae, alopecia cicatricial, alopecia areata ophiasis, alopecia areata diffusa, reticular alopecia areata, sisaipho alopecia areata, congenital alopecia, drug-induced alopecia, alopecia mucinosa, alopecia marginalis, androgenetic alopecia, traction alopecia and syphilitic alopecia.

The term "cucurbitaceae family" as used herein is taken to consist of approximately 125 genera and 960 species, mainly in tropical and subtropical regions. All species are sensitive to frost. Most of the plants in this family are annual vines, but there are also woody lianas, thorny shrubs, and trees (Dendrosicyos). Many species have large, yellow or white flowers. The stems are hairy and pentangular. Tendrils are present at 90° to the leaf petioles at nodes. Leaves are exstipulate alternate simple palmately lobed or palmately compound. The flowers are unisexual, with male and female flowers on different plants (dioecious) or on the same plant (monoecious). The female flowers have inferior ovaries. The fruit is often a kind of modified berry called a pepo.

The term "Ecballium elaterium" (L.) ("squirting cucumber" or "donkey's green") as used herein is taken to be a perennial plant from the family Cucurbitaceae, a Mediterranean medicinal plant that has been investigated for several of its pharmacological properties. Ecballium elaterium has a large fleshy root, from which several round, thick, rough stems rise, branching and trailing like the common cucumber, but without tendrils. The leaves are petiolate, large, rough, irregularly cordate, and of a grayish-green color. The flowers are yellow and axillary. The fruit has the shape of a small oval cucumber, about an inch and a half long and one inch thick. It has a greenish or grayish color, and is covered with stiff hairs or prickles. When fully ripe, it separates from the peduncle, and throws out its juice and seeds with considerable force through an opening at the base, where it was attached to the footstalk. The name of squirting cucumber was derived from this circumstance, and the scientific and official title (elaterium) is supposed to have had a similar origin, though some authors maintain that the term elaterium was applied to the drug rather from the mode of its operation upon the bowels than from the projectile property of the fruit. The word elaterium was used by Hippocrates to signify any active purge.

The term "leaching process for the extraction of terpenes" as used herein is taken to be a solid-liquid extraction from the raw material (i.e. a plant portion of ecbalium elaterium) using as extractant a non-polar liquid phase capable of solubilising terpenes (compounds of non-polar nature).

### Detailed description

The applicant has now surprisingly discovered that a composition comprising cucurbitacins and/or their glucoside derivatives, wherein cucurbitacins D, I, L and A and their glucoside derivatives are present, as a whole, in terms of percentage by weight, in an amount greater than 25% of the total weight of the cucurbitacins and their glucoside derivatives, and wherein cucurbitacins B, E and the derivative of cucurbitacin E, ecballic acid, are each present at a concentration below 2.5% of the total weight of the cucurbitacins and their glucoside derivatives, is highly effective in compositions for the treatment and prevention of hair loss.

In order to demonstrate the above, the applicant has prepared a series of vegetable extracts by using 5 g of the root of Ecbalium elaterium as raw material for the leaching process detailed in example 1. These vegetable extracts contained a total content of cucurbitacins and their glucoside derivatives, as detailed in example 2, in the range from 200 mg/L to 1 g/L; wherein the most concentrated cucurbitacins detected in the extracts were the D, I, L and A forms, which constituted approximately, as a whole expressed as percentage by weight, an amount ranging from 25 to 50% of the total weight of the cucurbitacins and their glucoside derivatives. Other cucurbitacins such as cucurbitacin O, P, C, H, J, F and Q were also present at lower concentrations (each of these cucurbitacins were present in an amount greater than 2.5 weight percent of the total weight of the cucurbitacins and their glucoside derivatives).

However, surprisingly cucurbitacins B, E and the derivative of cucurbitacin E, ecballic acid, which are the most concentrated cucurbitacins or derivatives found in Ecballium elaterium, were detected at trace levels (concentration below 2.5% of the total weight of the cucurbitacins and their glucoside derivatives) or not detected at all.

These vegetable extracts were used by the applicant to prepare the shampoo composition illustrated in example 3. This shampoo composition was tested, alone or in combination with other treatments, on patients suffering from different types of alopecia as illustrated in examples 4 to 8. The results show that these types of compositions are highly effective for the treatment and prevention of hair loss.

Accordingly, the first aspect of the present invention provides a composition (composition of the invention) comprising cucurbitacins and their glucoside derivatives, wherein cucurbitacins D, I, L and A and their glucoside derivatives are present, as a whole, in terms of percentage by weight in an amount greater than 25% of the total weight of the cucurbitacins and their glucoside derivatives, and wherein cucurbitacins B, E and the derivative of cucurbitacin E, ecballic acid, are each present at a concentration below 2.5% of the total weight of the cucurbitacins and their glucoside derivatives.

The composition of the invention preferably comprises cucurbitacins D, I, L and A and their glucoside derivatives, as a whole, as percentage by weight, in an amount greater than 30% of the total weight of the cucurbitacins and their glucoside derivatives, more preferably in an amount greater than 35%, 40% or 45%.

In a more preferred embodiment of the present invention, cucurbitacins D, I, L and A and their glucoside derivatives are present, as a whole, as percentage by weight in an amount between 25 and 50% of the total weight of the cucurbitacins and their glucoside derivatives.

In a still more preferred embodiment of the invention, the composition of the invention further comprises:
a. Cucurbitacin O;
b. Cucurbitacin P;
c. Cucurbitacin C;
d. Cucurbitacin H;
e. Cucurbitacin J;
f. Cucurbitacin F; or
g. Cucurbitacin Q;
wherein each of these compounds is present in an amount greater than 2.5% of the total weight of the cucurbitacins and their glucoside derivatives.

Additionally, the applicant has discovered the presence of phenolic compounds such as hydroxytyrosol, tyrosol, flavonoids (luteolin, apigenin), simple phenols (vanillic acid, vanillin), secoiridoids (3,4-DHPEA-EDA, 3,4-DHPEA-EA, p-HPEA-EDA, p-HPEA-EDA) and other triterpenic compounds characteristic of olive oil (oleanolic acid and maslinic acid) in the vegetable extracts obtained in example 1. The antioxidant capacity of olive phenols enhances the biological activity of cucurbitacins and glucoside derivatives improving their therapeutic effect.

Thus, in a further embodiment, the composition of the invention further comprises phenols or phenolic compounds, preferably phenols or phenolic compounds obtained from olive oil.

A second aspect of the present invention refers to a vegetable extract obtained from Ecbalium Elaterium wherein said vegetable extract comprises cucurbitacins and/or their glucoside derivatives, wherein cucurbitacins D, I, L and A and/or their glucoside derivatives are present, as a whole, in terms of percentage by weight, in an amount greater than 25% of the total weight of the cucurbitacins and their glucoside derivatives, and wherein cucurbitacins B, E and the derivative of cucurbitacin E, ecballic acid, are each present at a concentration below 2.5% of the total weight of the cucurbitacins and their glucoside derivatives.

In a preferred embodiment of this aspect of the invention, the vegetable extract contains, before dilution, a total content of cucurbitacins and their glucoside derivatives in the range from 200 mg/L to 1 g/L. In a more preferred embodiment of the invention, the vegetable extract further comprises phenols or phenolic compounds, preferably phenols or phenolic compounds obtained from olive oil. More preferably, the vegetable extract comprises olive oil.

A third aspect of the invention refers to a composition (from hereinafter composition of the third aspect of the invention) comprising cucurbitacins and/or their glucoside derivatives, wherein cucurbitacins D, I, L and A and their glucoside derivatives are present, as a whole, in terms of percentage by weight, in an amount greater than 25% of the total weight of the cucurbitacins and their glucoside derivatives, and wherein cucurbitacins B, E and the derivative of cucurbitacin E, ecballic acid, are each present at a concentration below 2.5% of the total weight of the cucurbitacins and their glucoside derivatives, wherein this composition is obtainable by a leaching process comprising the steps of:
c. Obtaining a plant portion of a plant pertaining to the *cucurbitaceae* family and
d. extracting the vegetable cucurbitacins from said portion by a heating process and by using as an extractant a non-polar liquid phase capable of solubilising said compounds.

In a preferred embodiment of this aspect of the invention, the plant portion pertaining to the *cucurbitaceae* family is Ecballium elaterium.

In another embodiment of this aspect of the invention, the heating process is conducted at a temperature from 120 °C to 180 °C, preferably at a temperature of approximately 150 °C.

In another embodiment of this aspect of the invention, the plant portion is selected from the group consisting of: roots, stems, leaves or fruits or any combination thereof. Preferably the plant portion is a root.

In a still further embodiment of this aspect of the invention, the extractant comprises phenols or phenolic acids, preferably the extractant is olive oil, more preferably the extractant is enriched with natural or added content of antioxidants.

A fourth aspect of the invention refers to the composition of any of the previous aspects for its use in therapy.

A fifth aspect of the invention refers to the composition of any of the previous aspects for its use in the treatment or prevention of hair loss. Disorders and conditions that can result in hair loss include hereditary and congenital alopecia, hypotrichosis, trichotillomania, tinea capitis, telogen effluvium, anagen effluvium, monilethrix, trichorrhexis nodosa (trichonodosis), infections, which involve the hair or scalp such as mycotic infection, infestations involving the hair or scalp, endocrine conditions such as hyperthyroidism, hypothyroidism and diabetes mellitus, lupus, hormone imbalance, for example as a result of polycystic ovary syndrome, and sebaceous cysts.

Preferably, the composition is for use in the treatment or prevention of alopecia or hypotrichosis.

It has been reported that reduced production of androgens, estrogens and epidermal growth factor (EDG) can be useful in the treatment and prevention of hair loss. Accordingly, steroids which block or inhibit a hormone or factor associated with hair loss can optionally be added to the composition of any of the previous aspects. As an example, 5[alpha]-reductase inhibitors block the conversion of testosterone to the active 5[alpha]-dihydrotesterone (DHT) form and have been used in the treatment of hair loss disorders. Suitable steroids for the reduced production and inhibition of these compounds and factors include testolactone, pregnenolone, dehydroepiandrosterone (DHEA) diosgenin, spironolactone, finasteride and tamoxifen.

Indole-based compounds such as indole, melatonin, N-[2-(5-methoxy-1H-indol-3-yl)ethyl]acetamide, skatole and indole-3-carbinol; certain bioflavenoids such as quercetin methyl chalcone, and anti-inflammatory fatty acids such as TES TRIOLATE, or PX-13 have also demonstrated utility in the treatment of hair loss and accordingly these compounds may be added to the composition of any of the previous aspects.

Anti-fungal materials have also demonstrated utility in the treatment of hair loss. Accordingly, the composition of any of the previous aspects may comprise an anti-fungal material. Suitable antifungal materials include azole antifungal agents such as ketoconazole, miconazole, clotrimazole, econazole, bifonazole, butoconazole, fenticonazole, isoconazole, oxiconazole, sertaconazole, sulconazole, tioconazole, fluconazole, itraconazole, isavuconazole, ravuconazole, posaconazole, voriconazole and terconazolepolyene; polyene antifungal agents such as natamycin, rimocidin, filipin, nystatin, amphotericin B and candicin; allylamines such as terbinafine, amorolfine, naftifine and butenafine; echinocandin antifungal agents such as anidulafungin, caspofungin and micafungin, and other known antifungal agents such as benzoic acid in combination with a keratolytic agent, ciclopirox, flucytosine, griseofulvin, gentian violet haloprogintolnaftate, undecylenic acid and tea tree oil. The azole antifungal agents are of particular utility since they also inhibit the synthesis of testosterone, which function can also be useful in the treatment or prevention of hair loss conditions and disorders as described above.

Anti-inflammatory agents are effective at reducing inflammation and pain and may have a secondary effect on hair loss disorders. Anti-inflammatory agents suitable for use in the composition of any of the previous aspects include, but are not limited to, 2-arylpropionic acids (profens), salicylates, arylalkanoic acids, N-arylanthranilic acids (fenamic acids), pyrazolidine derivatives, oxicams, COX-2 inhibitors, sulphonanilides and other known anti-inflammatory agents such as licofelone and omega-3 fatty acids. Most preferably ibuprofen is comprised as the anti-inflammatory agent.

Antipruritic agents or constituents that provide a mild analgesic may be added to the composition to reduce associated side effects such as irritation or itching. Such compositions include, but are not limited to zinc oxide (calamine), methol, phenol and camphor.

Preferably, the composition is suitable for topical administration to a patient. The compositions contemplated by this invention include compositions adopted for topical application to the human scalp and/or skin. Conventional composition forms for this purpose include ointments, lotions, pastes, jellies, gels, mousses, sprays, foams, aerosols, powders and similar known composition forms.

Therefore, the composition may also comprise a suitable carrier or diluent. Suitable carriers or diluents may be aqueous and/or alcoholic and may include a viscous base to retain in situ the composition in use. Suitable diluents for use as carriers to form a lotion include water and lower alcohols or polyols, such as methanol, ethanol, isopropanol, glycerol or propylene glycol. To form a cream or ointment a paraffinic fraction and an emulsion base may be used. The carrier may also comprise other conventional carriers or diluents, for example, glucose, lactose, corn starch, starch paste, gum acacia, gelatin, mannitol, magnesium trisilicate, potato starch, urea, keratin or colloidal silica.

The term "ointment" includes creams having oleaginous water soluble emulsion bases, for example lanolin, petrolatum, glycols, glycerin and similar.

The compounds may be liposomal preparations or liquid emulsions or be dissolved in conventional solvents such as acetonitrile, dimethylformamide (DMF), dimethylacetamide (DMA), or alcohols such as propanol and similar.

Optionally, the composition may be adapted for oral administration to a patient. Conventional composition forms for this purpose include tablets, coated tablets, caplets, troches, lozenges, dispersions, suspensions and capsules and similar known composition forms.

Alternatively, the composition may be adapted for parenteral administration. Conventional routes of parental administration include intravenous, intramuscular, intralesional, intraarterial, subcutaneous, intradermal, transdermal, transmucosal and inhalational and similar known routes. Preferably, the composition is administered by intramuscular or intralesional routes.

The composition may further comprise at least one component selected from the group consisting of a mineral supplement, a vitamin supplement, essential oils, fragrances, colouring agents, preservatives and a skin absorption enhancer.

Suitable mineral supplements for use in the composition include, but are not limited to, iron, zinc, copper, magnesium and calcium and combinations thereof.

Suitable vitamin supplements for use in the composition include, but are not limited to, vitamin A (retinol), B group vitamins such as vitamin B1 (thiamine), vitamin B2 (riboflavin), vitamin B3 (niacin), vitamin B5 (pantothenic acid), vitamin B6 (pyridoxine pyridoxal or pyridoxamine), B7 (Biotin), B9 (folic acid) and B12 (cobalamin), vitamin C (ascorbic acid), vitamin D (calciferol), vitamin E (tocopherol) and vitamin K (phyllochinone) and combinations thereof.

Suitable essential oils for use in the composition include, but are not limited to, jasmine oil, tea tree oil and citrus oils.

Suitable fragrances for use in the composition include menthol, benzyl alcohol, eugenol, phenoxyethanol, isopropyl palmitate, isopropyl myristate, benzyl salicylate, phenylethyl salicylate, thymol, isoamyl salicylate, triton X-100 surfactant, benzoic acid, benzyl benzoate, methyl salicylate, phenol, oleic acid, caproic acid or carbaryl.

Conventional colouring agents suitable for use in the composition include, but are not limited to, tartrazine, quinoline yellow, sunset yellow, amaranth, ponceau 4R, erythrosine, red 2G, allura red AC, patent blue V, indigo carmine, brilliant blue FCF, fast green FCF, green S and iron oxides.

Suitable preservatives for use in the composition include, but are not limited to, parabens such as methyl and propyl paraben, sorbic acid, potassium sorbate, quaternium-15, methylchloroisothiazolinone, and iodopropynyl butylcarbamate (IPBC) and natural preservatives such as citrus oils.

The composition may additionally comprise a skin absorption enhancer to facilitate absorption of the composition through the skin of the patient. Suitable skin absorption enhancers for this purpose include pentane 1,5-diol, N-dodecyl-2-pyrrolidone and its acetate analogue, fatty acids such as oleic acid, terpenes, esters such as isopropyl myristate, khellin and khellin analogues, methyl nicotinate, MSM-decylmethylsulfoxide, diethylene glycol, citric acid, pyruvic acid, phenoxyethanol, transcutol, phosphatidyl choline, a medium chain triglyceride oil (MCT oil) and water.

Administration of the composition to the affected area of a patient may be carried out in any manner which will result in delivery of an effective amount of the composition to the affected area of the patient. Preferably, the composition is applied topically to the affected area of the patient, which may be the scalp or another body surface. Preferably, the composition is administered topically to the affected area or areas between one and three times daily, and most preferably twice daily.

Preferably, the composition in the form of a shampoo may be used daily for treatment of the condition either alone, or in combination with the topical administration.

Thus, a sixth aspect of the present invention refers to a shampoo composition (from hereinafter shampoo composition of the invention) comprising:
c. A cleansing base; and
d. Active ingredients,
wherein at least one of the active ingredients comprises the composition as defined in any the previous aspects.

The cleansing base is constituted by surphactants preferably of the types alkyl sulphates and/or an alkyl ether sulphates.

The term "active ingredients" are the conditioning agents whose roles are to provide care for and beautification of the hair, and the specific active ingredients to mitigate and correct an imbalance affecting the scalp such as the composition of the invention.

In a preferred embodiment, the shampoo composition of the invention further comprises any of the following components or any combination thereof:
f. Thickeners for surfactant preparation;
g. Pearlescents and opacifiers;
h. Perfumes;
i. Colorants; or
j. Stabilizing agents.

In a further embodiment, the shampoo composition of the invention is an anti-dandruff composition comprising active ingredients capable of re-establishing the natural microbial flora on the skin surface.

In another embodiment, the shampoo composition of the invention is a shampoo for greasy hair comprising a cleansing base capable of eliminating excess sebum.

In yet another embodiment, the shampoo composition of the invention is for sensitive scalp and comprises mild anionic surphactants and amphoterics and/or non-ionic surphactants.

In a still further embodiment, the shampoo composition of the invention is for dry hair and comprises:
d. A fat absorber; and/or
e. An abrasive to dislodge soil; and/or
f. An alkalizer

The frequency of the application as well as the application time may be varied according to the individual objective. For example, the hair loss composition of the invention may be applied as a shampoo which is immediately rinsed out or may be applied as a lotion, shampoo, ointment or gel which is applied to the affected area and left to penetrate into the skin for a period of time. The ointment formulations are particularly suitable for use on facial areas such as the eyebrows or beard, whereas the spray formulations are preferred for diffuse types of hair loss such as male or female pattern hair loss or telogen effluvium. Gel and lotion formulations are suitable for all types of hair loss. Liposome formulations may be used for all types of hair loss, but are particularly suitable in cases where the patient is devoid of any existing hair, for example severe cases of alopecia areata, alopecia totalis and alopecia universalis.

In each case, it is recommended that the hair treatment composition is used on a regular basis and for a period of time to meet the specified objective of prevention of hair loss and/or stimulation of regrowth.

An eighth aspect of the present invention refers to the shampoo of the invention or to the lotion of the invention, for its use in the treatment or prevention of hair loss.

The following examples serve to illustrate the present invention; these examples are in no way intended to limit the scope of the invention.

### Examples

### Example 1: Preparation of the composition of the invention.

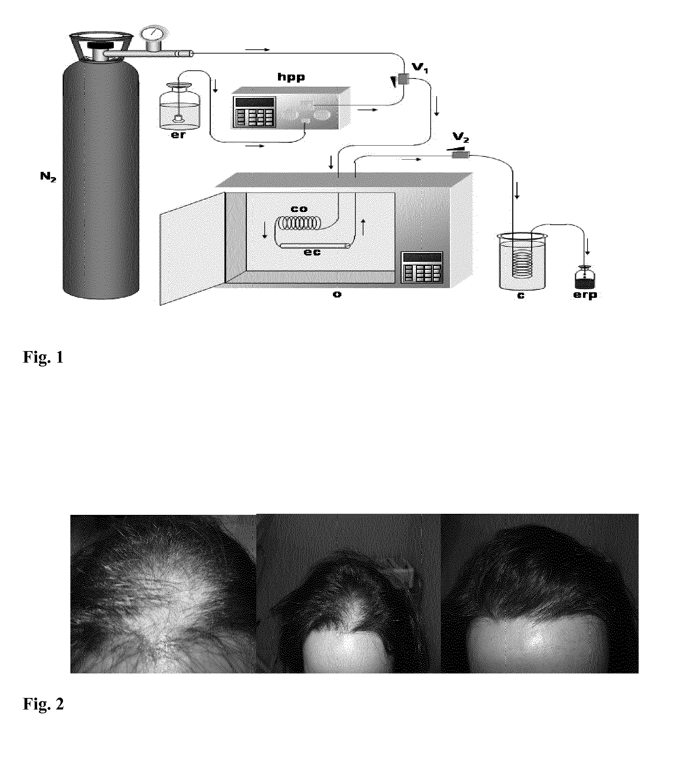

The procedure is based on the extraction system shown in Fig. 1 in which 5 g of the root of Ecbalium Elaterium was placed in the extraction cell **ec**. The cell was assembled, as the scheme shows, and located in the oven **o**. The cell was filled with extra virgin olive oil (EVOO) as extraction solvent with the high-pressure pump (EVOO volume 20 mL). The system was pressurized with 10 bar by opening the inlet valve **V₁** and closing the restrictor valve **V₂** to assure the liquid state of the extractant. Subsequently, the temperature in the oven was increased to the working temperature (150 °C) and kept under these conditions for 15 min. Then, valve **V₁** was switched to allow N₂ to push the extract, which was cooled in coil **c** and finally collected in **erp.**

### Example 2: Analysis of different extracts obtained as illustrated in example 1

The total content of cucurbitacins and their glucoside derivatives in the **erp** extracts, as measured by liquid chromatography-mass spectrometry using a high accuracy time of flight mass analyzer (limit of detection of the method: 0.5-1.0 mg/L), was determined to be in the range from 200 mg/L to 1 g/L.

On average the most concentrated cucurbitacins in the extracts were the D, I, L and A forms, which constituted approximately, as a whole, from 25 to 50% of the total weight of the cucurbitacins and their glucoside derivatives. Other cucurbitacins such as cucurbitacin O, P, C, H, J, F and Q were also present at lower concentrations (each of these cucurbitacins were present in an amount greater than 2.5% of the total weight of the cucurbitacins and their glucoside derivatives). However, it is worth pointing out that cucurbitacins B, E and the derivative of cucurbitacin E, ecballic acid, which are the most concentrated cucurbitacins or derivatives in vegetal material obtainable from Ecballium elaterium, were detected at trace levels (concentration below 2.5% of the total weight of the cucurbitacins and their glucoside derivatives, in this specific example below 5 mg/L of the total volume of the extract) or not detected in the extracts with the method used here due to hydrolysis during the leaching process.

### Example 3: Preparation of 250 mL of the shampoo composition of the invention.

The ingredients used for the preparation of 250 mL of the shampoo composition of the invention were:

### Example 3.a. Neutral shampoo

7% Ether lauryl sodium sulphate
5% Cocamidopropyl betaine
1.5% Sulfosuccinic acid
0.15% polyquatemium-10#
0.4% Extract obtained in example 1.

### Example 3.b. Antiseborrheic shampoo

7.5% Ether lauryl sodium sulphate
5.5% Cocamidopropyl betaine
1.25% Sulfosuccinic acid
0.15% polyquatemium-10#
0.5% Zinc pyrithione
0.4% Extract obtained in example 1.

In both cases, the ingredients were mixed by stirring in order to obtained a homogenous solution, which was thus ready for application.

### Example 4: Treatment of alopecia caused by hormone deregulations.

A woman with alopecia attributed to a post-partum hormone imbalance was treated with the shampoo composition of example 3a.

In this case the treatment was carried out three times per week for a period of one year, with daily use of the recommended shampoo. Each treatment session involved:
a. Cleaning the head skin with ozone vapour for 5 min.
b. Washing with the shampoo of example 3a.
c. Stimulation of blood circulation in the affected zone by acupuncture (needle hits).
d. Absorption favoured by application of a low-frequency current.
e. Washing with the shampoo of example 3a.

Figure 2 shows the initial state, evolution after treatment for 6 months, and final results after one year treatment.

The results illustrate that after one-year of treatment the affected zone was completely recovered.

### Example 5: Treatment of androgenetic alopecia or alopecia of seborrheic origin.

Androgenetic alopecia or alopecia of seborrheic origin is the most common type of alopecia affecting the masculin gender. In this case, the patient suffered from seborrhea with deterioration of the capilar ramifications. The treatment in this case consisted in a chemical peeling by using ethanol containing 1% iodine and cristallized mint in ozone vapour. The objective of this first step was to removed epidemial layers and renew them with higher quality layers. After this step, the treatment was similar to the one exemplified in the previous example, namely:
- Washing with the shampoo of example 3b.
- Stimulation of blood circulation in the affected zone by acupuncture (needle hits).
- Absorption favoured by application of a low-frequency current.
- Washing with the shampoo of example 3b.

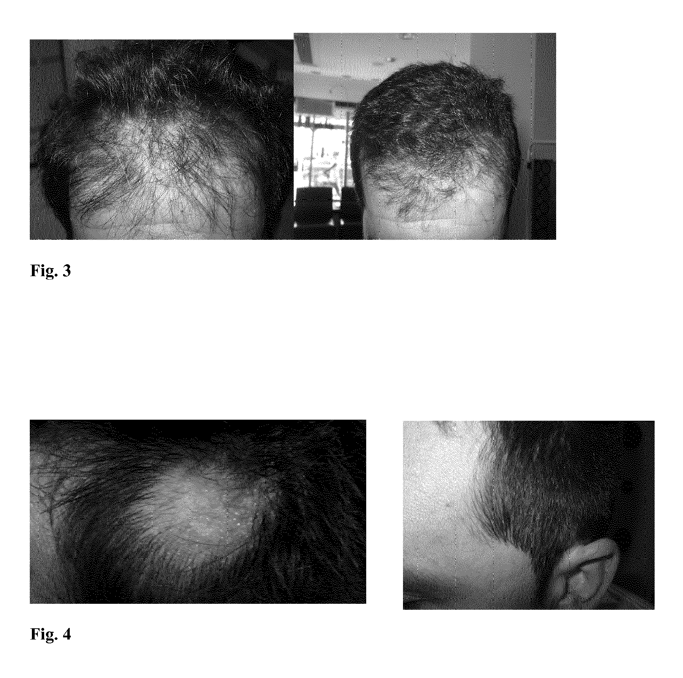

Figure 3 illustrates the results before treatment and after treatment for a period of 6 months.

As shown, the patient experienced substantial hair recovery after treatment.

### Example 6: Treatment of alopecia areata.

The affected zone from a man suffering from alopecia areata was treated two times per week for a period of two months. The treatment was as follows:
- Cleaning the head skin with ozone vapour for 5 min.
- Washing with the shampoo of example 3a.
- Stimulation of blood circulation in the affected zone by acupuncture (needle hits).
- Absorption favoured by application of a low-frequency current.
- Washing with the shampoo of example 3a.

Figure 4 illustrates the results before and after treatment.

As shown, hair regeneration was achieved after treatment.

### Example 7: Treatment of seborrheic dermatitis androgenic alopecia

In this example a man suffering from seborrheic dermatitis androgenic alopecia having significant hair loss (> 100 hairs / day) was treated with the shampoo composition of example 3b.

During the first week of treatment involved washing the hair with the shampoo composition of example 3b twice daily, leaving the shampoo for 5 minutes before rinsing. During the second week of treatment, the used of the shampoo composition was limited to once per day.

After the second week of treatment we observed a significant reduction in hair loss (< 80 hairs /day). Treatment was maintained for 6 months, observing 25% hair regeneration.

### Example 8: Treatment of pityriasis rosea

In this example, a man suffering from pityriasis rosea was treated with the shampoo composition of example 3a. As with example 7 the treatment involved washing the hair with the shampoo composition of example 3a twice daily, leaving the shampoo for 5 minutes before rinsing. During the second week of treatment, the used of the shampoo composition was limited to once per day. The treatment was maintained for 6 months, observing 50% hair regeneration.

## Claims

1. A composition comprising cucurbitacins and their glucoside derivatives, wherein cucurbitacins D, I, L and A and their glucoside derivatives are present, as a whole, in terms of percentage by weight in an amount greater than 25% of the total weight of the cucurbitacins and their glucoside derivatives, and wherein cucurbitacins B, E and the derivative of cucurbitacin E, ecballic acid, are each present at a concentration below 2.5% of the total weight of the cucurbitacins and their glucoside derivatives.

2. The composition of claim 1, wherein cucurbitacins D, I, L and A and their glucoside derivatives, as a whole, are present in amounts greater than 35% of the total weight of the cucurbitacins and their glucoside derivatives.

3. The composition of claim 1, wherein cucurbitacins D, I, L and A and their glucoside derivatives, as a whole, are present in amounts from 25 to 50% of the total weight of the cucurbitacins and their glucoside derivatives.

4. The composition of any one of claims 1 to 3, wherein the composition further comprises:
a. Cucurbitacin O;
b. Cucurbitacin P;
c. Cucurbitacin C;
d. Cucurbitacin H;
e. Cucurbitacin J;
f. Cucurbitacin F; and
g. Cucurbitacin Q;
wherein each of these compounds is present in an amount greater than 2.5% of the total weight of the cucurbitacins and their glucoside derivatives.

5. The composition of anyone of claims 1 to 4, wherein the composition further comprises olive oil.

6. An Ecbalium Elaterium vegetable extract which comprises a composition as defined in any of the previous claims.

7. The composition of anyone of claims 1 to 4, wherein the composition is obtainable by a leaching process comprising the steps of:
h. Obtaining a plant portion of a plant pertaining to the *cucurbitaceae* family and
i. extracting the vegetable cucurbitacins from said portion by a heating process and by using as an extractant a non-polar liquid phase capable of solubilising said compounds.

8. The composition of claim 7, wherein the plant portion pertaining to the *cucurbitaceae* family is Ecballium elaterium.

9. The composition of any one of claims 7 or 8, wherein the heating process is conducted at a temperature from 120 °C to 180 °C.

10. The composition of any one of claims 7 to 9, wherein the plant portion is a root.

11. The composition according to any one of claims 7 to 10, wherein the extractant is olive oil.

12. A shampoo composition which comprises:
a. A cleansing base; and
b. Active ingredients,
wherein at least one of the active ingredients comprises the composition as defined in any of claims 1 to 11.

13. A hair lotion comprising the composition as defined in any of claims 1 to 11.

14. The composition of any one of claims 1 to 11, for its use in therapy.

15. The composition of any one of claims 1 to 11 or the shampoo composition of claim 12 or the hair lotion of claim 13, for its use in the treatment or prevention of hair loss.

## Patentansprüche

1. Zusammensetzung, die Cucurbitacine und deren Glykosid-Derivate umfasst, wobei Cucurbitacine D, I, L und A und deren Glykosid-Derivate insgesamt in Gewichtsprozent in einer Menge größer als 25% des Gesamtgewichtes der Cucurbitacine und deren Glykosid-Derivate vorliegen, und wobei Cucurbitacine B, E und das Derivat von Cucurbitacin E, Ecballinsäure, jeweils in einer Konzentration von unter 2,5% des Gesamtgewichtes der Cucurbitacine und deren Glykosid-Derivate vorliegen.

2. Zusammensetzung nach Anspruch 1, wobei Cucurbitacine D, I, L und A und deren Glykosid-Derivate insgesamt in Mengen größer als 35% des Gesamtgewichtes der Cucurbitacine und deren Glykosid-Derivate vorliegen.

3. Zusammensetzung nach Anspruch 1, wobei Cucurbitacine D, I, L und A und deren Glykosid-Derivate insgesamt in Mengen von 25 bis 50% des Gesamtgewichtes der Cucurbitacine und deren Glykosid-Derivate vorliegen.

4. Zusammensetzung nach einem der Ansprüche 1 bis 3, wobei die Zusammensetzung ferner:
a. Cucurbitacin O;
b. Cucurbitacin P;
c. Cucurbitacin C;
d. Cucurbitacin H;
e. Cucurbitacin J;
f. Cucurbitacin F; und
g. Cucurbitacin Q;
umfasst, wobei jede dieser Verbindungen in einer Menge größer als 2,5% des Gesamtgewichtes der Cucurbitacine und deren Glykosid-Derivate vorliegt.

5. Zusammensetzung nach einem der Ansprüche 1 bis 4, wobei die Zusammensetzung ferner Olivenöl umfasst.

6. *Ecballium elaterium -* Pflanzenextrakt, der eine Zusammensetzung nach einem der vorhergehenden Ansprüche umfasst.

7. Zusammensetzung nach einem der Ansprüche 1 bis 4, wobei die Zusammensetzung durch ein Auslaugungsverfahren erhältlich ist, das folgende Schritte umfasst:
h. Erhalten eines Pflanzenteils einer der *Cucurbitaceae*-Familie angehörenden Pflanze, und
i. Extrahieren der pflanzlichen Cucurbitacine dieses Teils mittels eines Erwärmungsverfahrens und indem als Extrationsmittel eine nicht-polare flüssige Phase verwendet wird, die in der Lage ist, diese Verbindungen aufzulösen.

8. Zusammensetzung nach Anspruch 7, wobei der der *Cucurbitaceae-*Familie angehörende Pflanzenteil *Ecballium elaterium* ist.

9. Zusammensetzung nach einem der Ansprüche 7 oder 8, wobei das Erwärmungsverfahren bei einer Temperatur von 120 °C bis 180 °C durchgeführt wird.

10. Zusammensetzung nach einem der Ansprüche 7 bis 9, wobei der Pflanzenteil eine Wurzel ist.

11. Zusammensetzung nach einem der Ansprüche 7 bis 10, wobei das Extraktionsmittel Olivenöl ist.

12. Haarwaschmittelzusammensetzung, umfassend:
a. eine Reinigungsgrundlage; und
b. Wirkstoffe,
wobei mindestens einer der Wirkstoffe die Zusammensetzung nach einem der Ansprüche 1 bis 11 umfasst.

13. Haarlotion, welche die Zusammensetzung nach einem der Ansprüche 1 bis 11 umfasst.

14. Zusammensetzung nach einem der Ansprüche 1 bis 11, für dessen Verwendung in der Therapie.

15. Zusammensetzung nach einem der Ansprüche 1 bis 11 oder Haarwaschmittelzusammensetzung nach Anspruch 12 oder Haarlotion nach Anspruch 13, für dessen Verwendung bei der Behandlung oder der Vorbeugung gegen Haarausfall.

## Revendications

1. Composition comprenant des cucurbitacines et leurs dérivés glucosidiques, dans laquelle les cucurbitacines D, I, L et A et leurs dérivés glucosidiques sont présents, dans leur ensemble, en termes de pourcentage en poids dans une quantité supérieure à 25 % du poids total des cucurbitacines et de leurs dérivés glucosidiques, et dans laquelle les cucurbitacines B, E et le dérivé de cucurbitacine E, l'acide ecballique, sont chacun présents à une concentration inférieure à 2,5 % du poids total des cucurbitacines et de leurs dérivés glucosidiques.

2. Composition de la revendication 1, dans laquelle des cucurbitacines D, I, L et A et leurs dérivés glucosidiques, dans leur ensemble, sont présents dans des quantités supérieures à 35 % du poids total des cucurbitacines et de leurs dérivés glucosidiques.

3. Composition de la revendication 1, dans laquelle des cucurbitacines D, I, L et A et leurs dérivés glucosidiques, dans leur ensemble, sont présents dans des quantités comprises entre 25 et 50 % du poids total des cucurbitacines et de leurs dérivés glucosidiques.

4. Composition de l'une quelconque des revendications 1 à 3, dans laquelle la composition comprend en outre :
a. Cucurbitacine O ;
b. Cucurbitacine P ;
c. Cucurbitacine C ;
d. Cucurbitacine H ;
e. Cucurbitacine J ;
f. Cucurbitacine F ; et
g. Cucurbitacine Q ;
dans laquelle chacun de ces composés est présent dans une quantité supérieure à 2,5 % du poids total des cucurbitacines et de leurs dérivés glucosidiques.

5. Composition de l'une quelconque des revendications 1 à 4, dans laquelle la composition comprend en outre de l'huile d'olive.

6. Extrait végétal *d'Ecballium elaterium* qui comprend une composition telle que définie dans l'une quelconque des revendications précédentes.

7. Composition de l'une quelconque des revendications 1 à 4, dans laquelle la composition est obtenable par un procédé de lixiviation comprenant les étapes de :
h. Obtention d'une portion de plante d'une plante appartenante à la famille des *Cucurbitaceae* et
i. extraction des cucurbitacines végétales de ladite portion par un procédé de chauffage et en utilisant, comme agent d'extraction, une phase liquide non polaire capable de solubiliser lesdits composés.

8. Composition de la revendication 7, dans laquelle la portion de plante appartenante à la famille des *Cucurbitaceae* est *l'Ecballium elaterium.*

9. Composition de l'une quelconque des revendications 7 ou 8, dans laquelle le procédé de chauffage est réalisé à une température comprise entre 120 °C et 180 °C.

10. Composition de l'une quelconque des revendications 7 à 9, dans laquelle la portion de plante est une racine.

11. Composition selon l'une quelconque des revendications 7 à 10, dans laquelle l'agent d'extraction est de l'huile d'olive.

12. Composition de shampoing qui comprend :
a. Une base nettoyante ; et
b. Des ingrédients actifs,
dans laquelle au moins l'un des ingrédients actifs comprend la composition telle que définie dans l'une quelconque des revendications 1 à 11.

13. Lotion capillaire comprenant la composition telle que définie dans l'une quelconque des revendications 1 à 11.

14. Composition de l'une quelconque des revendications 1 à 11, pour son utilisation en thérapie.

15. Composition de l'une quelconque des revendications 1 à 11 ou la composition de shampoing de la revendication 12 ou la lotion capillaire de la revendication 13, pour son utilisation dans le traitement ou la prévention de la perte des cheveux.
